# EUROPEAN PATENT APPLICATION

(11) **EP 4 212 131 A1**
(43) Date of publication of application: **19.07.2023**
(21) Application number: 22151596.8
(22) Date of filing: 14.01.2022
(51) Int. Cl.: A61F 2/28, A61F 2/30, B33Y 80/00, G06N 3/00, A61B 17/80

(54) **A METHOD AND A COMPUTER PROGRAM FOR PERFORMING SCAFFOLD DESIGN OPTIMIZATION TOWARDS ENHANCED BONE HEALING, AND A METHOD TO MANUFACTURE A SCAFFOLD**

(71) Applicant: Perier-Metz Camille, 67201 Eckbolsheim (FR); Duda, Georg, 12209 Berlin (DE); Checa, Sara, 13353 Berlin (DE)
(72) Inventor: Perier-Metz Camille, 67201 Eckbolsheim (FR); Duda, Georg, 12209 Berlin (DE); Checa, Sara, 13353 Berlin (DE)
(74) Representative: Schulz Junghans Patentanwälte PartGmbB

(57) **Abstract**

The invention concerns a method, in particular a computer-implemented method, for performing scaffold design optimization (h) towards enhanced bone healing, the method comprising the steps of:
a) Inputting a set of parameters comprising information about a geometry and a material of a scaffold (3) to a computational model, the computational model comprising a finite element model and a mechano-biological computational model, wherein the finite element model determines and provides a set of mechanical information data for the set of parameters to the mechano-biological computational model, wherein the mechano-biological computational model determines from the set of mechanical information data a regenerated bone volume;
b) Determining a plurality of regenerated bone volumes for a plurality of sets of parameters according to step a), such as to generate a scaffold bone data associating the regenerated bone volume with the corresponding set of parameters;
c) Determining a computational surrogate model consisting of an analytical expression configured to associate the regenerated bone volumes with the sets of parameters from the scaffold bone data; and
d) Determining from the computational surrogate model an optimum set of parameters for which the computational surrogate model puts out a maximum regenerated bone volume.

The invention also concerns a computer program and a method to manufacture a scaffold (3).

## Description

The invention concerns a method and a computer program for performing scaffold design optimization towards enhanced bone healing, and a method to manufacture a scaffold which is designed using the steps of the method for performing the scaffold design.

Biomaterial scaffolds can be used as a guiding structure to promote bone regeneration in regions where a large piece of bone is missing. In order to achieve successful bone regeneration, cells need to be provided with the appropriate mechanical environment within the scaffold pores to produce the required extracellular matrix. Cellular behaviour within the scaffold pores is influenced by both mechanical and biological conditions, as disclosed by Cipitria et al., 2012 and Pobloth et al., 2018, which in turn change over the course of the bone regeneration process.

Although scaffold design has mainly relied on a trial and error approach so far, recently several research groups have adopted a more systematic approach using numerical or computational optimization methods to maximize or minimize specific properties, e.g. maximum stiffness; maximum permeability for good nutrient flow, as disclosed by Guest and Prévost 2006, Almeida and da Silva Bártolo 2010, Chen et al. 2011, Xiao et al. 2012, Dias et al. 2014, Wang et al. 2016, Langelaar 2016, Uth et al. 2017 and Metz et al. 2020. However, maximizing scaffold mechanical properties might not ensure a better bone regeneration, as too stiff constructs have been shown to achieve lower bone regeneration, as disclosed by Pobloth et al. 2018 and Reznikov et al. 2019. Nonetheless, building a scaffold mimicking the missing tissue cannot ensure to best support endogenous bone regeneration and implant osseointegration, as disclosed by Petersen et al. 2018.

Scaffolds can be designed to reach the same mechanical properties as the host tissue, as disclosed by Chang et al., 2017, Grbic and Comaniciu, 2018, Hollister et al., 2003, 2002 and Sturm et al., 2010, or withstand certain fatigue requirements, as disclosed by Pasini et al., 2014. However, this does not ensure that the right mechanical environment is reached within the scaffold pores. Another design strategy is to ensure the right mechanical properties within the scaffold pores immediately post-implantation, as disclosed by Boccaccio et al., 2019, 2018 and Percoco et al., 2020.

Bone regeneration is a highly dynamic process where different types of tissues are formed, remodelled and resorbed in specific locations over the regeneration period, creating a mechanical environment that changes over time. This changing mechanical environment in turns further influences tissue formation. A study conducted on spine fusion devices suggests that optimized design for the situation immediately after surgery would not yield optimal bone formation later, as disclosed by Bashkuev et al. 2015. An in-silico comparison between bone regeneration outcome for scaffolds optimized for the situation immediately after surgery versus one that takes the healing process into account reached the same conclusion in 2-D, as disclosed by Wu et al. 2021.To identify key design features of 3D scaffolds that support the bone defect regeneration is challenging since biomaterial scaffolds are usually not used alone but in combination with bone grafting or BMP-2 to increase the success rate, as disclosed by Viateau et al., 2007 and Pobloth et al., 2018. It is thought that cells (pre-osteoblasts and/or osteoprogenitors) that may be derived from a bone graft contribute to healing. Further, bone grafts have osteoconductive (guiding new bone ingrowth) and osteoinductive (enhancing bone deposition) properties, as disclosed by Finkemeier, 2002. However, how these different biologically stimulatory properties contribute alone or in combination to support the overall healing process remains poorly understood.

The numerical methods disclosed above frequently suffer from high computational costs that render the optimization process slow and cumbersome. Optimizations may take several days to weeks, which in a commercial setting is prohibitively long.

The problem to be solved by the present invention is to provide a method and a computer program that allows for an accurate and nonetheless fast scaffold design optimization targeting to enhance bone healing in combination with a method to manufacture such scaffolds in a fashion that enhances bone healing.

This problem is solved by a method for performing scaffold design optimization towards enhanced bone healing according to claim 1, a computer program according to claim 13, and method to manufacturing a scaffold according to claim 14. Advantageous embodiments of the method for performing scaffold design optimization towards enhanced bone healing are given in the dependent claims 2 to 12.

A first aspect of the invention relates to a method, in particular a computer-implemented method, for performing scaffold design optimization towards enhanced bone healing, e.g., in the case of a broken bone, especially where a piece of the broken bone is missing. The method comprises a first step of inputting to a computational model a set of parameters that comprise information about a geometry and information about a material of a scaffold. The inputting of a set of parameters can be conducted by selecting a set of parameters from a database, or another external source, such as a cloud service/system, or a plurality of external sources. Further, the set of parameters can be inputted manually by means of a data input device. The set of parameters can be selected and then provided to the computational model.

The computational model may be stored on a non-transitory storage medium of a computer and executed by at least one processor of the computer.

The set of parameters may be comprised, derived from or associated to image data acquired with a medical imaging apparatus configured to perform magnetic resonance imaging, positron emission tomography, x-ray imaging, such a CT.

The computational model comprises a finite element model and a mechano-biological computational model. The computational model is a method comprising a finite element model and a mechano-biological computational model. The finite element model can be built based on the geometry of the scaffold and the loading conditions. The finite element model determines, by means of a calculation or a finite element analysis, and provides a set of mechanical information data, which correspond to the inputted set of parameters, to the mechano-biological computational model, wherein the mechano-biological computational model determines from the set of mechanical information data a regenerated bone volume. The finite element model is a method that determines, by means of a calculation or a finite element analysis, and provides a set of mechanical information data, which correspond to the inputted set of parameters, to the mechano-biological computational model, wherein the mechano-biological computational model is a method that determines from the set of mechanical information data a regenerated bone volume.

The mechano-biological computational model can be an agent-based model, as for example disclosed by Perier-Metz et al., 2020. Furthermore, one of the models or methods based on partial differential equations can also be used as the mechano-biological computational model. In the following a brief description of the agent-based model is provided.

The agent-based model comprises a method that describes the individual cellular behavior based on stochastic processes and on mechanical information data, particularly a mechano-biological stimulus computed from an output resulting from the calculations performed by the finite element model. The agent-based model is based on a 3D regular matrix or a 3D- grid, for example with a spacing of 100µm, where each point on the grid may be described and represented by an agent, the point can be occupied by at most one cell of one of the following phenotypes: mesenchymal stromal cell "MSC", osteoblast "bone cell", chondrocyte "cartilage cell" or fibroblast "fibrous tissue cell". Each agent therefore may represent a single cell of one phenotype and the corresponding tissue which is associated to the phenotype, wherein the corresponding tissue can be granulation tissue, bone, cartilage or fibrous tissue, respectively.

Furthermore, for example if the distance between agents on the grid is larger than an expected cell size, then a single agent might as well comprise more than one cell as well as the corresponding tissues associated with the respective cells. Therefore, both cells and tissues may be represented in the agent-based model.

At an initial time point, that represents a time of scaffold implantation and a beginning of the healing process, in the agent-based model, only agents representing MSCs are considered in the simulation.

The agents representing MSCs can migrate at a given speed, such as 30 µm/h on the grid. This migration process is implemented as an iterative random walk process, i.e., each new position the agents adopt during an iteration step is randomly chosen among the free adjacent positions of the agent's current position, which represents the corresponding cell's current position. Additional constraints can be implemented into the agent-based model, such as surface guidance of the migration. In that case, a new position can be accessed by an agent only if the position is adjacent to previously deposited or intact tissue or a scaffold surface.

At each iteration step of the computational model, which can correspond to one healing day, a predefined fraction, e.g. 30% of the MSCs differentiate. The agents comprised by the fraction of MSCs that differentiate are changed to a different phenotype according to the mechano-biological stimulus. The different phenotype may be selected from the group of an osteoblast, a chondrocyte, a fibroblast or nothing for a stimulus favoring bone, cartilage, fibrous tissue and resorption, respectively. Because the agent may include the cell type and the corresponding tissue, the differentiation step directly leads to tissue deposition in the same position. Additional constraints can be implemented, such as surface guidance of the tissue deposition process. In that case, an agent representing MSC can differentiate only if it is adjacent to previously deposited or intact tissue or a scaffold surface.

Lastly, agents representing each of the cell phenotypes can proliferate or undergo apoptosis with given rates and depending on the local stimulus. The agents for which the local stimulus is favorable can proliferate, i.e. a new agent representing a new daughter cell of the same phenotype in an adjacent position is created, while the others can undergo apoptosis, that is the corresponding agent representing the corresponding cell would be removed from the agent position.

Depending on the exact set-up of the computational model comprising the finite element model and the mechano-biological computational model comprising the agent-based model, that is scaffold material, additional therapy such as growth factors, the cellular activity levels, i.e. proliferation, differentiation and apoptosis rates and migration speed, are changed to reflect a lack of biological stimulation corresponding to a large bone defect or an improved one, which can be due to a beneficial material or additional therapy.

The output of this mechano-biological computational model, that is for example the agent-based model, can be a volume of regenerated bone after a time interval in a given region of interest, for instance in the scaffold pores, corresponding to the expected time for healing or at least no more evolution of the defect's situation. This value is computed by adding all agents containing an osteoblast "bone" at the end of the simulation or computation of the mechano-biological computational model and multiplying those by a size of one agent of a predefined size. This volume can be divided by the total free volume, for example excluding the scaffold, within the region of interest to obtain a bone volume proportion.

Further, an alternative output of the mechano-biological computational model can be a computation of the time needed to achieve bone healing. Here, the bone healing has to be defined, for instance in terms of a predefined bone volume, which can be obtained from a database of medical data comprising a bone volume corresponding to a particular bone with a corresponding defect, or the continuity of the regenerated bone in the defect.

The output of the of the mechano-biological model may be provided from the processor of the computer to the non-transitory storage medium and may further be displayed on a display for graphical visualization of the output.

In a second step of the inventive method, a plurality of regenerated bone volumes for a plurality of sets of parameters according to the first step is determined, such as to generate a scaffold bone data associating the regenerated bone volume with the corresponding set of parameters, particularly for which the computational model has calculated the regenerated bone volume. The second step can be conducted or computed in parallel or series. In the case of a parallel computation of the second step, respective regenerated bone volumes for each of the corresponding set of parameters of the plurality of sets of parameters are determined according to the first step, all at once. However, in the case of a series computation, one regenerated bone volume for one of the set of parameters is determined according to the first step at a time, that is each of the corresponding regenerated bone volume for each of the corresponding set of parameters of the plurality of sets of parameters is determined one after the other in a sequential manner.

A third step of the inventive method is determining a computational surrogate model comprising a parametric model configured to associate the regenerated bone volumes with the sets of parameters from the scaffold bone data. The computational surrogate model being a method comprising the parametric model. The parametric model can be in the form of a polynomial function, which can be obtained by an interpolation method or a curve fitting method associating the regenerated bone volume as a function of the set of parameters from the scaffold bone data. The parametric model can be in the form of gaussian function as well. This is of advantage, because such a parametric model is computationally inexpensive and fast as compared to a computation of a regenerated bone volume for a set of parameters using the aforementioned computational model comprising the finite element model and the mechano-biological computational model.

A fourth step of the inventive method comprises determining from the computational surrogate model an optimum set of parameters for which the computational surrogate model puts out a maximum regenerated bone volume. This is of advantage because, this enables a determination of the scaffold design with the optimum set of parameters for an enhanced bone healing. For example, the fourth step of the inventive method can be performed using a computational optimization model, wherein the computational optimization model is a method that optimizes an input-output function globally, the input-output function is the numerical expression obtained from the third step.

The inventive method enables an optimization of scaffolds, for example 3D-printed scaffolds, towards enhanced bone regeneration. The method includes the bone regeneration outcome to optimize scaffold design, and not only an initial mechanical stimulus or initial mechanical properties. In addition, the use of the computational surrogate model allows for a set-up that can be run in a reasonable amount of time on standard computing machines. As such, the method steps that are computer-executable may be executed on the at least one processor of the computer. Further, due to the complexity and sheer amount of data that has to be handled and computed by the processor, it may be of advantage to use an array of parallel processors to execute the computations necessary to perform the method steps.

It is to be understood, that additional intermediate steps, which would be obvious to a person skilled in the art could be performed between any of the two consecutive steps of the inventive method.

According to an embodiment of the invention, each set of parameters can further comprise an information on patient-specific data, wherein the patient-specific data comprises a bone geometry acquired from a medical image of the patient and loading boundary conditions for the bone geometry. The bone geometry can comprise the broken bone geometry, for example where a piece of bone is missing. The loading boundary conditions for the bone geometry can be obtained from a patient-specific medical data. The loading boundary conditions can be applied based on estimation of typical loads under walking conditions, usually related to the weight of the patient. Furthermore, the loading boundary conditions may depend on the location of the broken bone, that is depending on the part of the body where the breakage of the bone has occurred. Based on which the type of forces such as compression loading forces, bending loading forces, torsion loading forces or a combination thereof can be applied as the corresponding loading boundary conditions.

Particularly, said patient-specific data may comprise or maybe derived from the image data acquired with the medical imaging apparatus.

According to an embodiment of the invention, the first step of the method can further comprise combining the bone geometry with the geometry of the scaffold and discretizing the bone geometry combined with the geometry of the scaffold into a computational mesh of the finite element model.

The finite element model can be based on the geometry of the defected bone to be treated with the scaffold. This combined geometry can be simplified, in particular with regards to the included intact bone extremities, for example simplified as circular or elliptical cylinders, or directly imported from imaging data, e.g., using an X-Ray image or a computer tomography "CT-scan". Furthermore, the combined geometry for the finite element model could preferably include intact bone extremities at least twice as long as the defect, the defect zone, that is where the scaffold will be implanted, including the fixation plate/ plates and screws according to the surgery planning, and the scaffold geometry.

This aforementioned combined geometry is discretized in a finite element software as a computational mesh. The computational mesh can comprise tetrahedral and/or hexahedral and/or polyhedral finite elements with appropriate precision.

The following loading boundary conditions can be implemented:
- One extremity of the bone, which is distal, can be clamped with no rotation and translation degrees of freedom
- On the other extremity, a load can be applied based on estimation of typical loads under walking conditions usually related to the weight of the subject patient. Mechanical properties can be attributed to each part of the body corresponding to the part with the broken bone, assuming either linear elastic behaviors of all tissues and materials, or poroelastic behaviors for the living tissues and linear elastic for the plate, screws and scaffold.

The different parts of the body can be either merged or tied to each other.

The mechano-biological computational model, that is for example the agent-based model, can be coupled with the finite element model in two ways. Firstly, the mechanical stimulus, that can be derived element-wise from the finite element analysis, can be used to influence cell behavior, and/or secondly, the tissue material properties can be updated in the finite element model at every iteration depending on the tissue distribution. More precisely, each element of the finite element model can be mapped to the agents it contains and its material properties can be defined as a weighted average of the tissues predicted in these agents, and the material properties can be further averaged over the last ten iterations to account for tissue deposition and maturation, as disclosed by Lacroix and Prendergast 2002. The finite element analysis and agent-based simulations can be run iteratively to predict the full regeneration process, wherein one iteration may represent the healing process of one day.

In other words, the finite element model can be built based on the geometry of the defect to be treated and on the weight of the patient corresponding to the loading conditions. Then, iteratively, a simulation or computation of the finite element model, i.e., a finite element analysis, can be conducted. From the simulation or computation of the finite element model, the local mechanics in each finite element corresponding to a region of the body of the patient, where the breakage of the bone has occurred, can be determined. These mechanical values can be used to compute a mechano-biological stimulus, such as a linear combination of octahedral shear strain and fluid relative velocity or thresholds on hydrostatic stress and minimal principal strain. Based on this stimulus, the cells implemented in the mechano-biological computational model, that is the agent-based model, could follow certain behaviors, such as differentiation, apoptosis or cell death, proliferation and migration. The cell distribution in the regenerating zone will be used to update the material properties in each finite element as each cell deposits a certain tissue type with distinct material properties.

The finite element analysis can then be run again and the whole process goes on iteratively, wherein one iteration can correspond to one healing day.

According to another embodiment of the invention, the computational model, particularly the mechano-biological computational model, calculates the regenerated bone volume as a function of time, wherein the computational model simulates, particularly iteratively simulates, cellular activity and mechanical environment changes, e.g., loading forces, over a course of healing for the bone regeneration. This embodiment allows consideration of the effects of dynamic changes on the bone regeneration over the course of healing for the bone regeneration as a function of time. The regenerated bone volume which will be used for the computational surrogate model in the third step of the inventive method, corresponds to the time of the end of the healing process.

Further, the set of parameters can comprise information about scaffold material degradation over time, wherein the computational model can, particularly iteratively, determine the regenerated bone volume in dependency of the scaffold material degradation. This would enable to incorporate a dynamic interaction between scaffold and bone regeneration, by means of the inclusion of the scaffold material degradation. The scaffold degradation can occur usually at longer time scales, such as after several months or years, as compared to the healing phase of the broken bone, which can be about a few weeks.

According to another embodiment of the invention, after the fourth step, the first step is executed again inputting the optimum set of parameters determined by the computational surrogate model, such that an optimum regenerated bone volume is calculated from the computational model, wherein if a difference between the optimum regenerated bone volume and the maximum regenerated bone volume obtained previously, from the computational surrogate model of the fourth step, is less than 5% of the optimum regenerated bone volume determined by means of the computational model using the optimum set of parameters, then the optimum set of parameters calculated in the fourth step is selected as the set of parameters for the scaffold design promoting enhanced bone healing.

In other words, after a determination of the optimum set of parameters for which the computational surrogate model puts out a maximum regenerated bone volume, the determined optimum set of parameters is inputted again to the computational model, wherein the computational model determines the regenerated bone volume. The simulations by the finite element model and mechano-biological computational model of the computational model are performed again to obtain the corresponding regenerated bone volume using the aforementioned optimum set of parameters. This regenerated bone volume obtained by the computations from the mechano-biological computational model corresponds to an optimum regenerated bone volume and is supposedly more accurate than the result from the computational surrogate model. To determine a degree of deviation of the optimum regenerated bone volume determined by the computational model, especially the mechano-biological computational model, and the computational surrogate model, the optimum regenerated bone volume determined by the mechano-biological computational model is compared to the determined maximum regenerated bone volume determined by the computational surrogate model. Upon comparison, if the difference is less than 5% of the optimum regenerated bone volume determined by means of the computational model using the optimum set of parameters, the optimum set of parameters calculated in fourth step, that is using the computational surrogate model, is selected as the set of parameters for the scaffold design promoting enhanced bone healing.

This is of advantage, as using the computational surrogate model enables a fast and computationally inexpensive determination of the optimum set of parameters resulting in a maximum regenerated bone volume. Further, performing a simulation using the determined optimum set of parameters by means of the finite-element and the mechano-biological computational model in addition, would increase the reliability of the obtained result using the computational surrogate model. Hence, this would enable the inventive method to be computationally efficient and accurate as a whole.

Furthermore, if the difference obtained by the comparison is more than 5% of the optimum regenerated bone volume determined by means of the computational model using the optimum set of parameters, then all the four steps of the inventive method are executed with more sets of parameters so as to obtain an improved computational surrogate model. This is done by obtaining a higher degree of sampling of the regenerated bone volume associated to the sets of parameters, i.e. a higher sampling of the parameter space is obtained and thus a higher sampled space for the regenerated bone volume. This allows increasing the accuracy of the computational surrogate model, thereby increasing the accuracy of the inventive method as whole.

The information about the geometry of the scaffold can comprise information about pore sizes, and/or pore size distribution, and/or curvature of the scaffold. Further, the information about the material of the scaffold can comprise information about a Young's modulus, and/or Poisson's ratio, and/or porosity, stiffness, and/or density of the material of the scaffold. It is further possible, that information about a material distribution of the scaffold is provided, for example where the Young's modulus, and/or Poisson's ratio, and/or porosity, and/or stiffness, and/or density is a variable at different regions within the scaffold geometry

According to another embodiment of the invention, the plurality of sets of parameters is selected using a uniform sampling scheme or a Latin hypercube sampling scheme.

The Latin hypercube sampling scheme is a method configured to generate a set of parameter values in a multidimensional space. For a size N, N corresponding to the number of values of each parameter in the multidimensional space, the multidimensional space is divided into sub-spaces obtained by dividing each side of the parameter space by N. Points are then placed randomly in various sub-spaces so as to have exactly one point in each row and column, which can be extended to further dimensions. This allows a sampling representing the variability of the design space and further provides an accurate sparse representation of the complete design space.

According to another embodiment of the invention, the computational surrogate model is a Multivariate Adaptive Regression Splines model (MARS) or a Kriging model.

A MARS model is a non-parametric regression method and can be represented as a product of hinge functions, such as piece-wise linear functions, which makes the resulting computational surrogate model to be easily interpretable. Further, piece-wise cubic functions can be employed to implement the MARS model as the computational surrogate model. The MARS model is built through adding terms to reduce the mean squared error the most until a certain tolerance level is reached, and then removing the terms that are least value-adding in the model to avoid over-fitting. The MARS model enables to provide results with a high accuracy.

The Kriging model is a type of Gaussian process regression method, wherein a weighted average of known points around a point of interest is computed. The definition of these weights can be based on assumptions made on co-variances. Different type of auto-correlations, known to a person skilled in the art, can be defined when applying the Kriging model, wherein assuming an exponential auto-correlation provides results with a high accuracy.

Further, the computational surrogate model can comprise surrogate models such as a neural network model, or a radial basis function model, or a support vector machine model, or a Gaussian process regression model.

According to another embodiment of the invention, for determining the optimum set of parameters yielding the maximum regenerated bone volume in the fourth step of the inventive method, a computational optimization model is used, wherein the computational optimization model is a computational optimization method that is selected from the group of a direct search algorithm, or a genetic algorithm, or a particle swarm optimization method.

The parametric model or an objective function based on the parametric model can comprise several local optima, hence, a global, gradient-free optimization approach can be adopted for the computational optimization model rather than a gradient-based optimization approach which may be known to a person skilled in the art. Hence, the computational optimization model can be selected from a group of gradient-free optimization approaches such as a direct search algorithm, or a genetic algorithm, or a particle swarm optimization method. These gradient-free optimization approaches or algorithms, when applied to the computational surrogate models, result in a faster evaluation of the maximum regenerated bone volume as compared to when applied to the mechano-biological computational model directly.

A second aspect of the invention is a computer program and/or a computer program product comprising instructions which, when the program is executed by a computer, cause the computer to carry out the steps of the inventive method for performing scaffold design optimization towards enhanced bone healing.

Particularly, the set of parameters may be provided by the computer program from the non-transitory storage medium of the computer, as elaborate din the context of the first aspect of the invention. Further, the non-transitory storage medium may comprise the image data and/or the patient-specific data. Image data and/or the patient specific data may be or may have been acquired by means of a medical imaging apparatus that is configured to record the image data and/or the patient specific data and to convert said data into digital data and to store or transmit said digital data representative of the patient specific data (and also referred to as such) to the computer comprising the non-transitory storage medium. This can be achieved e.g. by means of any network connection the medical imaging apparatus and the computer is connected to.

Further, the computer executing the computer program may be connected to a display that is configured to display any output of the of the computer program. A third aspect of the invention is a method for manufacturing a scaffold which is designed using the steps of the inventive method or computer program for performing scaffold design optimization towards enhanced bone healing, wherein the method to manufacture the scaffold is a 3D-printing method or an injection molding method. Particularly, the method of manufacturing comprises a step of generating a digital file that is configured and adapted to comprise instructions for an additive manufacturing device or system, such that the additive manufacturing device or system may produce the scaffold as computed by the method according to the invention.

The term '3D-printing' may be synonymously used for additive manufacturing methods.

Particularly, the method according to the first aspect may be extended such as to comprise steps of the method of manufacturing the scaffold. In case the method is incorporated in a computer program, the computer or the computer-system executing the computer program, may be connected or configured to transmit the scaffold design resulting from the execution of the method to an additive printing device or system, e.g. by means of converting the result in a file format adapted for being executed by an additive manufacturing device or system.

The term 'computerized device', 'computerized system' or a similar term denotes an apparatus comprising one or more processors operable or operating according to one or more programs.

The term 'computer' or system thereof, may be used herein as ordinary context of the art, such as a general-purpose processor or a micro-processor, RISC processor, or DSP, possibly comprising additional elements such as memory or communication ports. Optionally or additionally, the terms 'computer' or derivatives thereof denote an apparatus that is capable of carrying out a provided or an incorporated program and/or is capable of controlling and/or accessing data storage apparatus and/or other apparatus such as input and output ports. The term 'computer' denotes also a plurality of processors or computers connected, and/or linked and/or otherwise communicating, possibly sharing one or more other resources such as a memory inform of a non-transitory storage medium.

As used herein, the terms 'server' or 'client' or 'backend' denotes a computer or a computerized device providing data and/or operational service or services to one or more other computerized devices or computers.

The terms 'software', 'computer program', 'software procedure' or 'procedure' or 'software code' or 'code' or 'application' or 'app' may be used interchangeably according to the context thereof, and denote a product or a method comprising one or more instructions or directives or circuitry for performing a sequence of operations that generally represent an algorithm and/or other process or method. The program may be stored in or on a medium such as RAM, ROM, or disk, or embedded in a circuitry accessible and executable by an apparatus such as a processor or other circuitry.

The processor and program may constitute the same apparatus, at least partially, such as an array of electronic gates, such as FPGA or ASIC, designed to perform a programmed sequence of operations, optionally comprising or linked with a processor or other circuitry.

As used herein, without limiting, a process represents a collection of operations for achieving a certain objective or an outcome.

Similarly, a model may represent a collection of operations for achieving a certain objective or an outcome

The term 'configuring' and/or 'adapting' for an objective, or a variation thereof, implies using at least a software and/or electronic circuit and/or auxiliary apparatus designed and/or implemented and/or operable or operative to achieve the objective.

A device, such as a non-transitory storage medium, storing and/or comprising a computer program and/or data particularly constitutes an article of manufacture.

Unless otherwise specified, the program and/or data are stored in or on a non-transitory medium.

In the context of embodiments of the present disclosure, by way of example and without limiting, terms such as 'operating' or 'executing' imply also capabilities, such as 'operable' or'executable', respectively.

The invention is explained below using the embodiment examples shown in the enclosed drawings.

It is shown in
- Fig. 1:: a flow chart of an embodiment of the inventive method for performing scaffold design optimization towards enhanced bone healing,
- Fig. 2:: a schematic view of a finite element model geometry consisting of a bone geometry combined with a geometry of a scaffold,
- Fig. 3:: a schematical longitudinal view of a geometry of the scaffold shown in Fig. 2,
- Fig. 4: a schematical longitudinal view of a half geometry of the geometry of the scaffold shown in Fig. 3,
- Fig. 5: a top view of the geometry of the scaffold shown of Figs. 3 and 4, and
- Fig. 6: three-dimensional plot depicting the percentage of regenerated bone volume against set of input parameters comprising information of the pore sizes of the geometry of the scaffold shown in Fig. 2.

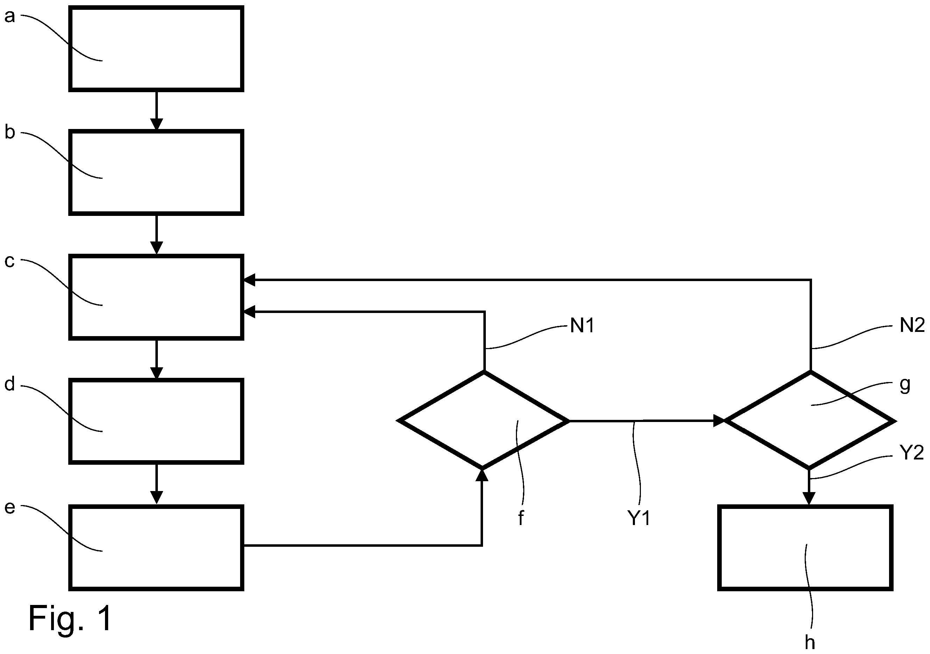

Fig. 1 shows a flow chart of an embodiment of the inventive method for performing scaffold design optimization towards enhanced bone healing. The method is a computer-implemented method. The method comprises a first step a) of initial sampling of a set of parameters comprising information about a geometry and a material of a scaffold and inputting the set of parameters to a computational model. The sampling of the parameters is obtained by Latin Hypercube Sampling technique "LHS". The LHS technique is disclosed by Simpson et al. 2002. The computational model comprises a finite element model and a mechano-biological computational model. The computational model computes a regenerated bone volume using the inputted set of parameters.

In the second step b), a plurality of regenerated bone volumes is determined for a plurality of sets of parameters according to first step a), such as to generate a scaffold bone data associating the regenerated bone volume with the corresponding set of parameters, for which the computational model has calculated the regenerated bone volume.

In a third step c) of the method, a computational surrogate model is determined consisting of a parametric model configured to associate the regenerated bone volumes with the sets of parameters from the scaffold bone data. A Kriging model is used as the computational surrogate model, which is implemented using the Matlab kriging toolbox DACE, as disclosed by Lophaven et al. 2002.

The method comprises a fourth step d), in which the computational surrogate model determines an optimum set of parameters for which the computational surrogate model puts out a maximum regenerated bone volume using a computational optimization model. The computer optimization model can for example be implemented using the Matlab Global optimization toolbox, as disclosed by The MathWorks, Inc. 2020.

The exemplary embodiment of the method comprises a fifth step e), in which the computational model calculates an optimum regenerated bone volume using the optimum set of parameters determined by the computational surrogate model in the fourth step d of the method.

In a sixth step f) of the exemplary method, a difference is calculated between the optimum regenerated bone volume obtained from the computational model in the fifth step e) and the maximum regenerated bone volume obtained from the computational surrogate model of the fourth step d). In this sixth step f), it is checked if the difference is less than 5% of the optimum regenerated bone volume determined by means of the computational model using the optimum set of parameters. If the difference is not less than 5% of the optimum regenerated bone volume, then the method reverts back to the third step c), as indicated by the first arrow N1. The steps c) to e) are executed with more sets of parameters so as to obtain an improved computational surrogate model by obtaining a higher degree of sampling of the regenerated bone volume associated to the sets of parameters.

Each repetition of the steps c) to e) is followed by the sixth step f) of the method, where every time it is checked by the method if the difference is less than 5% of the optimum regenerated bone volume determined by means of the computational model using the optimum set of parameters. The repetition of steps c) to f) is conducted until the difference is less than 5% of the optimum regenerated bone volume, in which case the method moves forward to a seventh step g), as indicated by the second arrow Y1.

In the seventh step g) of the method, it is checked if the optimum regenerated bone volume is better, for example higher, than all previously stored values. If the result of the checking is that the optimum regenerated bone volume is not better, for example higher, than all previously stored values, then the method reverts back to the third step c), as indicated by the third arrow N2. The steps c) to f), and when the conditions of the sixth step f) are met, followed by the seventh step g), are executed. These repetitions are conducted until it is found that the optimum regenerated bone volume is better, for example higher, than all previously stored values. In that case, the method finally determined the set of parameters for the final optimized scaffold design h) to obtain enhanced bone healing, as indicated by the fourth arrow Y2.

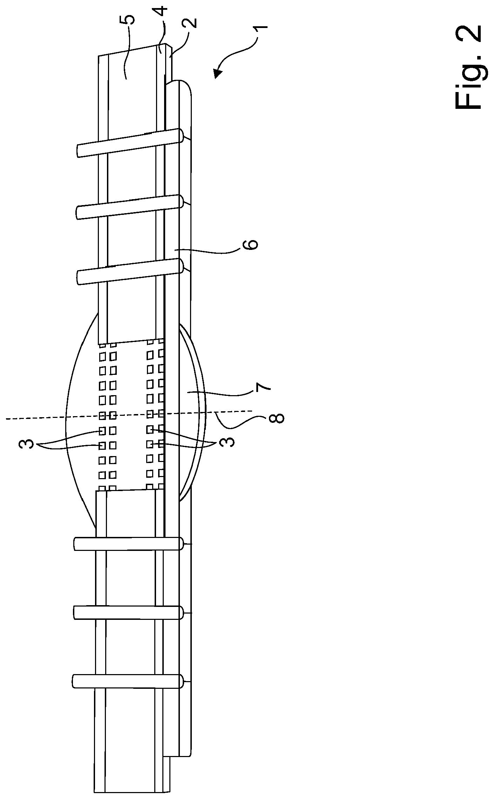

Fig. 2 depicts a sectional view of a finite element model geometry 1 for a simulation or computation using the finite element model, i.e. a finite element analysis wherein the finite element model geometry 1 is a geometry used in the finite element analysis. The finite element geometry 1 comprises a bone geometry 2 comprising a geometry of the cortical bone 4 and bone marrow 5. The finite element model geometry 1 further comprises the geometry of the scaffold 3, the steel support structure 6, which is also called a plate fixation system, and a granulation tissue 7 surrounding the geometry of the scaffold 3, bone geometry 2 and the steel support structure 6. The symmetry line 8 indicates that the finite element model geometry is symmetrical about the symmetry line 8, in this case the finite element simulation can be conducted considering only a half of the finite element model geometry 1 as boundary conditions corresponding to symmetry can be considered. This means the finite element simulations can be conducted considering only one half of the finite element model geometry 1, whereas the results of the simulated one half can be assumed to be the same for the other half of the finite element model geometry 1, the results being mapped as being reflected about the symmetry line 8.

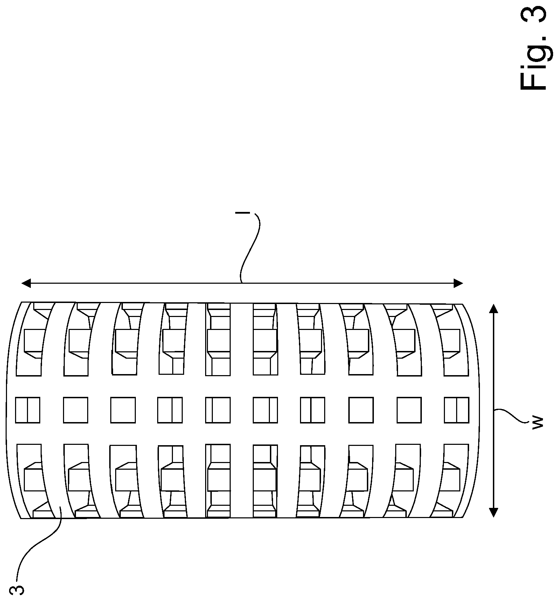

Fig. 3 shows a schematic longitudinal view of a geometry of the scaffold 3, the scaffold 3 has cylindrical shape with a length I along the longitudinal direction and width w, the width in this case corresponds to the diameter of the scaffold 3.

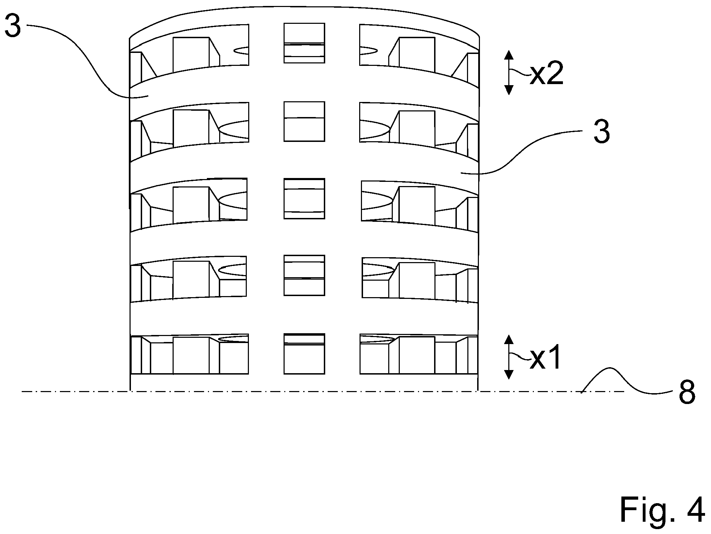

Fig. 4 shows a longitudinal view of a half geometry of the scaffold 3 of Fig. 3, assuming the symmetry condition as depicted by the symmetry line 8 in Fig. 2.

The pore on the end of the cylindrical scaffold 3 has a length x2, whereas the pore in the middle along the longitudinal direction, that is on the symmetry line 8, has a length x1. A linear distribution of the pore size from a length x1 to the length x2 in the longitudinal direction, that is over the half-length of the scaffold 3, which is I/2 is considered. The corresponding lengths of each pores distributed radially for a given distance from the symmetry line 8, are assumed to be uniform corresponding to the length of the pore size at that distance.

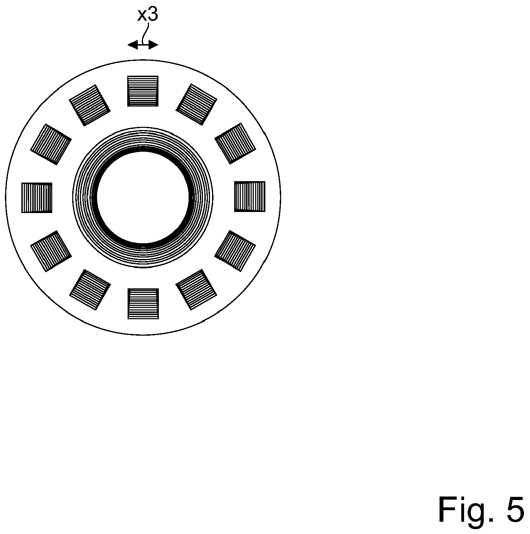

Fig. 5 shows a top view of the geometry of the scaffold 3 of figs. 3 and 4, wherein each pore has a length x3 along the circumferential direction.

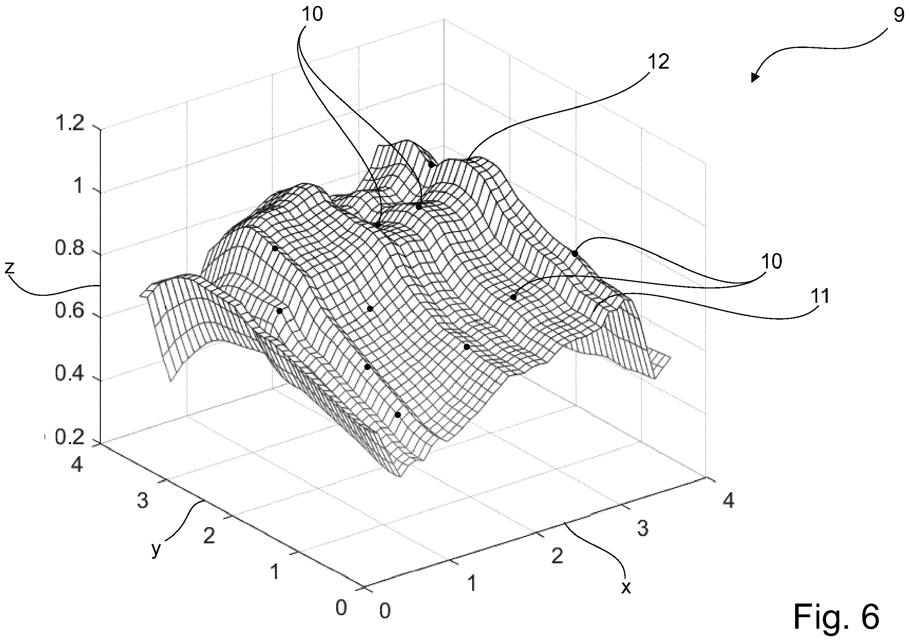

Fig. 6 depicts three-dimensional plot 9 depicting the percentage of regenerated bone volume along the z-axis z. The x-axis x denotes a distribution of the input parameters, where the length x1 in millimeters of the scaffold shown in Fig. 3 and 4 is varied. The y-axis y denotes a distribution of the input parameters, where the length x2 in millimeters of the scaffold 3 shown in Figs. 3 and 4 is varied.

As described by the first step a) and the second step b) of the method, a plurality of regenerated bone volumes for a plurality of sets of parameters according to first step a) is determined, such as to generate a scaffold bone data, denoted by the plurality of dots 10, associating the regenerated bone volume with the corresponding set of parameters, for which the computational model has calculated the regenerated bone volume. A plurality of dots 10 are plotted in the three-dimensional plot, each denoting a corresponding regenerated bone volume percentage corresponding to a set of parameters of the geometry of the scaffold 3 based on the lengths x1 and x2.

As described in the third step c) of the method, the computational surrogate model associates the regenerated bone volumes with the sets of parameters from the scaffold bone data and generates a parametrical model, here it is denoted by the surface 11.

In the fourth step d), the computational surrogate model determines an optimum set of parameters for which the computational surrogate model puts out a maximum regenerated bone volume, which is denoted by the peak 12 on the surface 11, using a computational optimization model.

**List of reference signs**

| | |
|---|---|
| Finite element model geometry | 1 |
| Bone geometry | 2 |
| Scaffold | 3 |
| Cortical bone | 4 |
| Bone marrow | 5 |
| Steel support structure | 6 |
| Granulation tissue | 7 |
| Symmetry line | 8 |
| Three-dimensional plot | 9 |
| Dot | 10 |
| Surface | 11 |
| Peak | 12 |
| First step | a |
| Second step | b |
| Third step | c |
| Fourth step | d |
| Fifth step | e |
| Sixth step | f |
| Seventh step | g |
| Optimized scaffold design | h |
| Length of the scaffold geometry | I |
| Width of the scaffold | w |
| X-axis | x |
| Y-axis | y |
| Z-axis | z |
| Length of the smallest pore | x1 |
| Length of the largest pore | x2 |
| Width of the pore | x3 |
| First arrow | N1 |
| Second arrow | Y1 |
| Third arrow | N2 |
| Fourth arrow | Y2 |

### References

Almeida H de A, da Silva Bártolo PJ (2010) Virtual topological optimisation of scaffolds for rapid prototyping. Medical Engineering & Physics 32:775-782. https://doi.org/10.1016/j.medengphy.2010.05.001
Bashkuev, M., Checa, S., Postigo, S., Duda, G., Schmidt, H., 2015. Computational analyses of different intervertebral cages for lumbar spinal fusion. J Biomech 48, 3274-3282. https://doi.org/10.1016/j.jbiomech.2015.06.024
Boccaccio, A., Fiorentino, M., Gattullo, M., Manghisi, V.M., Monno, G., Uva, A.E., 2019. Geometry Modelling of Regular Scaffolds for Bone Tissue Engineering: A Computational Mechanobiological Approach, in: Cavas-Martinez, F., Eynard, B., Fernández Cañavate, F.J., Fernandez-Pacheco, D.G., Morer, P., Nigrelli, V. (Eds.), Advances on Mechanics, Design Engineering and Manufacturing II, Lecture Notes in Mechanical Engineering. Springer International Publishing, pp. 517-526.
Boccaccio, A., Uva, A.E., Fiorentino, M., Bevilacqua, V., Pappalettere, C., Monno, G., 2018. A Computational Approach to the Design of Scaffolds for Bone Tissue Engineering, in: Piotto, S., Rossi, F., Concilio, S., Reverchon, E., Cattaneo, G. (Eds.), Advances in Bionanomaterials, Bionam 2016. Springer-Verlag Berlin, Berlin, pp. 111-117.
Chang, C.-C., Chen, Y., Zhou, S., Mai, Y.-W., Li, Q., 2017. Computational Design for Scaffold Tissue Engineering, in: Li, Q., Mai, Y.W. (Eds.), Biomaterials for Implants and Scaffolds. Springer-Verlag Berlin, Berlin, pp. 349-369.
Chen Y, Zhou S, Li Q (2011) Microstructure design of biodegradable scaffold and its effect on tissue regeneration. Biomaterials 32:5003-5014. https://doi.org/10.1016/j.biomaterials.2011.03.064
Cipitria, A., Lange, C., Schell, H., Wagermaier, W., Reichert, J.C., Hutmacher, D.W., Fratzl, P., Duda, G.N., 2012. Porous scaffold architecture guides tissue formation. Journal of Bone and Mineral Research 27, 1275-1288. https://doi.org/10.1002/jbmr.1589
Dias MR, Guedes JM, Flanagan CL, et al., 2014. Optimization of scaffold design for bone tissue engineering: A computational and experimental study. Medical Engineering & Physics 36:448-457. https://doi.org/10.1016/j.medengphy.2014.02.010
Finkemeier, C. G. (2002). Bone-grafting and bone-graft substitutes. J. Bone Joint Surg. 84, 454-464.
Guest JK, Prévost JH., 2006. Topology optimization of creeping fluid flows using a Darcy-Stokes finite element. International Journal for Numerical Methods in Engineering 66:461-484. https://doi.org/10.1002/nme.1560
Grbic, S., Comaniciu, D., 2018. Data driven framework for optimizing artificial organ printing and scaffold selection for regenerative medicine. US20180053346A1.
Hollister, S., Chu, G., Taboas, J., 2003. Design methodology for tissue engineering scaffolds and biomaterial implants. US20030069718A1.
Hollister, S.J., Maddox, R.D., Taboas, J.M., 2002. Optimal design and fabrication of scaffolds to mimic tissue properties and satisfy biological constraints. Biomaterials 23, 4095-4103. https://doi.org/10.1016/S0142-9612(02)00148-5
Langelaar M., 2016. Topology optimization of 3D self-supporting structures for additive manufacturing. Additive Manufacturing 12:60-70. https://doi.org/10.1016/j.addma.2016.06.010
Lophaven SN, Nielsen HB, Søndergaard J., 2002. DACE - A Matlab Kriging Toolbox, Version 2.0
Metz, C., Duda, G.N., Checa, S., 2020. Towards multi-dynamic mechano-biological optimization of 3D-printed scaffolds to foster bone regeneration. Acta Biomaterialia 101, 117-127. https://doi.org/10.1016/j.actbio.2019.10.029
Pasini, D., Khanoki, S.A., Tanzer, M., 2014. Bone replacement implants with mechanically biocompatible cellular material. US20140363481A1.
Percoco, G., Uva, A.E., Fiorentino, M., Gattullo, M., Manghisi, V.M., Boccaccio, A., 2020. Mechanobiological Approach to Design and Optimize Bone Tissue Scaffolds 3D Printed with Fused Deposition Modeling: A Feasibility Study. Materials 13, 648. https://doi.org/10.3390/ma13030648
Perier-Metz, C., Duda, G.N., Checa, S., 2020. Mechano-Biological Computer Model of Scaffold-Supported Bone Regeneration: Effect of Bone Graft and Scaffold Structure on Large Bone Defect Tissue Patterning. Front. Bioeng. Biotechnol. 8. https://doi.org/10.3389/fbioe.2020.585799
Petersen A, Princ A, Korus G, et al., 2018. A biomaterial with a channel-like pore architecture induces endochondral healing of bone defects. Nature Communications 9:4430. https://doi.org/10.1038/s41467-018-06504-7
Pobloth, A.-M., Checa, S., Razi, H., Petersen, A., Weaver, J.C., Schmidt-Bleek, K., Windolf, M., Tatai, AÁ., Roth, C.P., Schaser, K.-D., Duda, G.N., Schwabe, P., 2018. Mechanobiologically optimized 3D titanium-mesh scaffolds enhance bone regeneration in critical segmental defects in sheep. Science Translational Medicine 10, eaam8828. https://doi.org/10.1126/scitranslmed.aam8828
Reznikov N, Boughton OR, Ghouse S, et al., 2019. Individual response variations in scaffold-guided bone regeneration are determined by independent strain- and injury-induced mechanisms. Biomaterials 194:183-194. https://doi.org/10.1016/j.biomaterials.2018.11.026
Simpson T, Dennis L, Chen W, 2002. Sampling Strategies for Computer Experiments: Design and Analysis. International Journal of Reliability and Application 2:209-240
Sturm, S., Zhou, S., Mai, Y.-W., Li, Q., 2010. On stiffness of scaffolds for bone tissue engineering - a numerical study. J Biomech 43, 1738-1744. https://doi.org/10.1016/j.jbiomech.2010.02.020
The MathWorks, Inc. (2020) Global Optimization Toolbox manual. Natick, Massachusetts, United States
Uth N, Mueller J, Smucker B, Yousefi A-M., 2017. Validation of scaffold design optimization in bone tissue engineering: finite element modeling versus designed experiments. Biofabrication 9:015023. https://doi.org/10.1088/1758-5090/9/1/015023
Viateau, V., Guillemin, G., Bousson, V., Oudina, K., Hannouche, D., Sedel, L., et al., 2007. Long-bone critical-size defects treated with tissue-engineered grafts: a study on sheep. J. Orthop. Res. 25, 741-749. doi: 10.1002/jor.20352
Wu C, Fang J, Entezari A, et al., 2021. A time-dependent mechanobiology-based topology optimization to enhance bone growth in tissue scaffolds. Journal of Biomechanics 110233. https://doi.org/10.1016/j.jbiomech.2021.110233
Wang Y, Luo Z, Zhang N, Qin Q., 2016. Topological shape optimization of multifunctional tissue engineering scaffolds with level set method. Struct Multidisc Optim 54:333-347. https://doi.org/10.1007/s00158-016-1409-2
Xiao D, Yang Y, Su X, et al., 2012. Topology optimization of microstructure and selective laser melting fabrication for metallic biomaterial scaffolds. Transactions of Nonferrous Metals Society of China 22:2554-2561. https://doi.org/10.1016/S1003-6326(11)61500-8

## Claims

1. A method, in particular a computer-implemented method, for performing scaffold design optimization (h) towards enhanced bone healing, the method comprising the steps of:
a) Inputting a set of parameters comprising information about a geometry and a material of a scaffold (3) to a computational model, the computational model comprising a finite element model and a mechano-biological computational model, wherein the finite element model determines and provides a set of mechanical information data for the set of parameters to the mechano-biological computational model, wherein the mechano-biological computational model determines from the set of mechanical information data a regenerated bone volume;
b) Determining a plurality of regenerated bone volumes for a plurality of sets of parameters according to step a), such as to generate a scaffold bone data associating the regenerated bone volume with the corresponding set of parameters;
c) Determining a computational surrogate model comprising a parametric model configured to associate the regenerated bone volumes with the sets of parameters from the scaffold bone data; and
d) Determining from the computational surrogate model an optimum set of parameters for which the computational surrogate model puts out a maximum regenerated bone volume.

2. The method according to claim 1, wherein each set of parameters further comprises an information on patient-specific data, wherein the patient-specific data comprises a bone geometry (2) acquired from a medical image of the patient and loading boundary conditions for the bone geometry.

3. The method of claim 2, wherein step a) of the method further comprises combining the bone geometry (2) with the geometry of the scaffold (3) and discretizing the bone geometry (3) combined with the geometry of the scaffold (3) into a computational mesh of the finite element model.

4. The method according to claim 2 or 3, wherein the computational model calculates the regenerated bone volume as a function of time, wherein the computational model simulates cellular activity and mechanical environment changes over a course of healing for the bone regeneration.

5. The method according to claim 4, wherein the set of parameters comprises information about scaffold material degradation over time, wherein the computational model determines the regenerated bone volume in dependency of the scaffold material degradation.

6. The method according to any of the preceding claims, wherein after step d), step a) is performed inputting the optimum set of parameters determined by the computational surrogate model, such that an optimum regenerated bone volume is calculated from the computational model, wherein if a difference between the optimum regenerated bone volume and the maximum regenerated bone volume obtained from the computational surrogate model of step d) is less than 5% of the optimum regenerated bone volume determined by means of the computational model using the optimum set of parameters, then the optimum set of parameters calculated in step d) is selected as the set of parameters for the scaffold design promoting enhanced bone healing.

7. The method according to claim 6, wherein if the difference obtained by the comparison is more than 5% of the optimum regenerated bone volume determined by means of the computational model using the optimum set of parameters, then steps a) to d) are executed with more sets of parameters so as to obtain an improved computational surrogate model.

8. The method according to any of the preceding claims, wherein the information about the geometry of the scaffold (3) comprises information about pore sizes (x1, x2, x3), and/or pore size distribution, and/or curvature of the scaffold (3).

9. The method according to any of the preceding claims, wherein the information about the material of the scaffold comprises information about a Young's modulus, and/or Poisson's ratio, and/or porosity, and/or stiffness, and/or density of the material of the scaffold.

10. The method according to any of the preceding claims, wherein the plurality of sets of parameters are selected using a uniform sampling scheme or a Latin hypercube sampling scheme.

11. The method according to any of the preceding claims, wherein the computational surrogate model is a multivariate adaptive regression splines model, or a Kriging model.

12. The method according to any of the preceding claims, wherein for determining the optimum set of parameters yielding the maximum regenerated bone volume in step d) a computational optimization model is used, wherein the computational optimization model is selected from a group of a direct search algorithm, or a genetic algorithm, or a particle swarm optimization method.

13. A computer program comprising instructions which, when the program is executed by a computer, cause the computer to carry out the steps of the method of any one of preceding claims.

14. A method for manufacturing a scaffold which is designed using the steps of the method of any of the claims 1 to 12, wherein the method to manufacture the scaffold (3) is a 3D-printing method or an injection molding method.
